# EUROPEAN PATENT SPECIFICATION

(11) **EP 0 416 232 B1**
(45) Date of publication and mention of the grant of the patent: **16.08.1995**
(21) Application number: 90112426.3
(22) Date of filing: 29.06.1990
(51) Int. Cl.: A61K 31/505, A61K 47/10, A61K 47/20, A61K 47/18

(54) **Stable injectable pharmaceutical formulation for folic acid and leucovorin salts and method**
Stabile injizierbare pharmazeutische Formulation mit Folinsäure und Leukovorinsalze und Verfahren
Fomulation stable injectable à base d'acide folique et de sels de la leucovorine et méthode

(30) Priority: 21.08.1989 US 396573
(43) Date of publication of application: 13.03.1991
(73) Proprietor: AMERICAN CYANAMID COMPANY, Wayne, NJ 07470-8426 (US)
(72) Inventor: Haeger, Bruce Edwin, Highland Mills, New York 10930 (US)
(74) Representative: Wächtershäuser, Günter, Prof. Dr.

(56) References cited:
- WO-A-88/04927
- US-A- 2 695 860
- US-A- 4 071 620

## Description

The present invention relates to new improved injectable compositions comprising folic acid salts and leucovorin salts. More particularly, it relates to compositions comprising folic acid salts and leucovorin salts that remain stable for prolonged periods under normal storage conditions because they include tromethamine and monothioglycerol as a buffering agent/antioxidant combination.

### BACKGROUND OF THE INVENTION

Folic acid and its salts and leucovorin and its salts are known to be pharmaceutically effective. See, Remington's Pharmaceutical Sciences, Seventeenth Edition, Mack Publishing Co., Easton, PA 1985 (Remington's 17th Ed.) p. 1023. Folic acid is used to treat vitamin deficiencies. Cosulich, U.S. 2,688,018 describes the preparation of such compounds and their clinical use for controlling the toxicity of aminopterin and other antifolic acid compounds and as hematopoietic drugs. Active derivatives of such compounds are described in Shive, U.S. 2,741,608. In U.S. 4,500,711, Wisowaty et al., describe the purification of leucovorin and its salts. Kerwar et al., U.S. 4,746,662 disclose that the antiarthritic efficacy of methotrexate can be potentiated by injection of an aqueous solution of leucovorin or its salts. EPO Patent Publication No. 0,266,042, May 4, 1988, describes using pure leucovorin isomers to manufacture medicaments for methotrexate rescue, for treatment of colorectal cancer in combination with 5-fluorouracil, and for treating folate deficiency.

Both folic acid and leucovorin are only sparingly soluble in water. Therefore they are administered in the form of salts such as alkaline metal and alkaline earth metal salts, such as the sodium salt of folic acid and the calcium salt of leucovorin, the l-isomer of the latter being preferred.

The compound N-(4-(((2-Amino-1,4-Dihydro-4-oxo-6-pteridinyl)methyl)-amino)benzoyl)-L-glutamic acid, sodium salt (Sodium Folate) having the formula 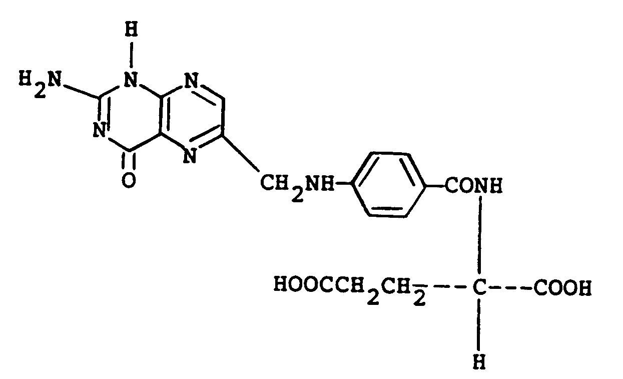
is used principally to stimulate specifically the production of red blood cells, white blood cells and platelets in persons suffering from certain megaloblastic anemias.

The compound N-(4-(((2-Amino-5-formyl-1,4,5, 6,7,8-hexahydro-4-oxo-6-pteridinyl)methyl)amino)benzoyl)-L-glutamic acid, calcium salt (1:1), pentahydrate, (Leucovorin Calcium USP) having the formula: 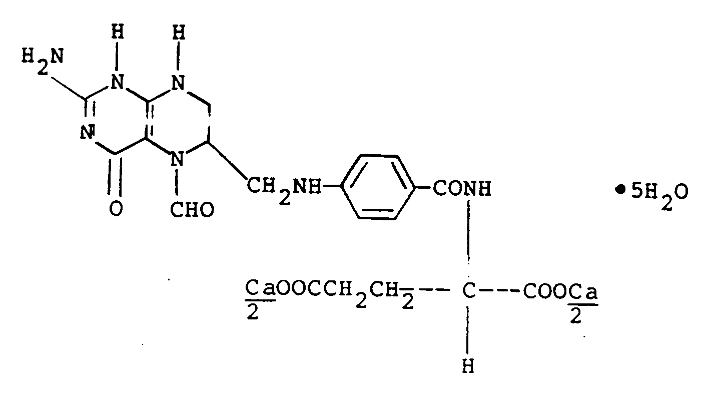
is used principally as an antidote for folic acid antagonists such as methotrexate, which block the conversion of folic acid into folinic acid. Merck Index, Tenth Edition, p. 603. Both folic acid and leucovorin salts are formulated in water for injection and may contain suitable preservatives, as described under Folvite® Injection and Leucovorin Calcium Injection in the Physician's Desk Reference, Medical Economics Company, Oradell, NJ 1989 (PDR) pp. 1120 and 1124, respectively.

Both compounds, but especially Leucovorin Calcium, require an alkaline pH of 7.7-8.2 for maximum stability. They are also light sensitive and prone to oxidative degradation in aqueous solution, thus requiring the use of amber glass for protection against photosensitivity and the use of nitrogen gas as a protectant throughout the bulk liquid manufacturing process and as a package headspace gas.

In the past, methyl and propyl parabens (p-hydroxy benzoates) were used as a preservative for such salts, especially Leucovorin Calcium Injection. However, the parabens were later found to have short term effectiveness at the alkaline pH required for maximum folic acid and/or leucovorin stability. Subsequently, benzyl alcohol was approved and widely used as the new preservative for the products. See "Folvite® Folic Acid Solution" and "Leucovorin Calcium Injection", PDR, pp. 1120 and 1124 respectively. Although benzyl alcohol is effective as a preservative, it lacks the mild buffering activity afforded by the parabens. As a consequence the pH of the products tend to be somewhat unstable, slowly drifting downward with time toward pH 6.5-7.0. This is below the above-mentioned optimum range and may require expiration dating to be very short term.

It has now been found that the factors of pH drift, nitrogen headspace variability and short term expiry dating can be overcome in accordance with this invention by using a buffer/antioxidant combination. The buffer comprises 2-Amino-2-(hydroxymethyl)-1, 3-propanediol,also known as tromethamine. The antioxidant comprises 3-Mercapto-1,2-propanediol, also known as monothioglycerol.

Both compounds, tromethamine and monothioglycerol, are toxicologically acceptable, and are described in U.S. Pharmacopeia XXI, U.S. Pharmacopeial Convention, Rockville, MD 1985 (U.S.P. XXI) at pages 1102 and 1580, respectively.

Surprisingly, the new buffer/antioxidant compositions of the present invention, for tromethamine and monothioglycerol, have been found to be superior to the presently used formulations, in terms of ability to control pH in the desired region of folic acid and leucovorin stability, and in their ability to retard oxidation, and therefore degradation, in sealed dosage forms. These new buffer/antioxidant compositions make it possible to extend the expiry dating of the product and provide the potential for development of a broader dosage line for such products, e.g. single or multi-dose vials containing greater volumes and higher concentrations of either folic acid or leucovorin salts than the present single dose (3-5 mg/ml) ampuls, such as a 10 mg/ml, 100 mg/vial dosage preparation. Unexpectedly also, benzyl alcohol becomes an optional ingredient and this is desirable expecially in cases where large doses are needed in emergencies and too much benzyl alcohol is not recommended.

### SUMMARY OF THE INVENTION

According to the present invention there are provided stable, injectable aqueous compositions comprising
(i) an effective amount of water-soluble pharmaceutically-acceptable salt of folic acid or leucovorin; optionally,
(ii) a small, effective preservative amount of benzyl alcohol; and
(iii) an effective amount of a buffer/antioxidant combination comprising (a) tromethamine and (b) monothioglycerol, said combination (iii) being present in an amount at least sufficient to maintain the pH of said composition in a predetermined range of from about 6 to about 10 and to protect the composition against degradation induced by oxygen or light.

In preferred embodiments, the compositions comprise those wherein said salt comprises a salt of dl-leucovorin; those wherein the compositions also include (iv) sodium chloride in an amount sufficient to render said composition isotonic; those which also include (v) a pH adjustor comprising an acid or a base in amount sufficient to adjust the pH to any value within said range; those wherein said pH adjustor comprises hydrochloric acid or sodium hydroxide. Especially preferred are compositions wherein said l-leucovorin salt comprises calcium leucovorin. Special mention is made of a formulation comprising from 3 mg/ml to 25 mg/ml of calcium leucovorin in an isotonic solution at a pH of from 6.5 to 8.5, with or without benzyl alcohol at a concentration of from 0.0% w/v to 0.909% w/v, TRIS (tromethamine) at a concentration of from 0.10% w/v to 0.30% w/v, monothioglycerol at a concentration of from 0.10% w/v to 0.30% w/v; sodium chloride at a concentration from 0.45% to 0.65% w/v, with hydrochloric acid at a concentration of from 5.0 v/v% to 10.0 v/v% and sodium hydroxide at a concentration of from 1.0 w/v% to 5.0 w/v% added as required to adjust the pH; and particularly those in which the concentration of calcium leucovorin on the one hand is 3.0 mg to 3.54 mg/ml and 10 mg to 11 mg/ml, on the other, and the concentrations of benzyl alcohol, TRIS (tromethamine), monothioglycerol and sodium chloride are from 0.765% w/v to 1.035% w/v, 0.150% w/v, 0.200% w/v and 0.560% w/v, respectively.

The present invention also contemplates a method for stabilizing compositions as above defined comprising adding thereto an effective amount of the buffer/antioxidant combination comprising tromethamine and monothioglycerol.

### DETAILED DESCRIPTION OF THE INVENTION

The injectable composition of this invention can be prepared by techniques well known to those skilled in the art of pharmaceutical formulation. The substantially pure salts may be prepared, mixed with the other components, filtered, filled into containers, and sealed under aseptic conditions.

Folic acid and its salts can be prepared by any convenient method, for example 2,3-dibromopropionaldehyde, dissolved in a water miscible organic solvent (alcohol, dioxane), is added to a solution of equal molecular quantities of 2,4,5-triamino-6-hydroxypyrimidine and p-aminobenzyl-glutamic acid, maintaining a pH of about 4 by the controlled addition of alkali as the reaction progresses. If sodium hydroxide is used as the alkali, sodium folate is obtained. To make calcium leucovorin, folic acid is, for example simultaneously hydrogenated and formylated in 90 to 100% formic acid under the influence of platinum oxide catalyst to yield leucovorin. Conversion to the calcium salt may be accomplished by dissolving the leucovorin in sodium hydroxide solution, treating the calcium chloride, and precipitating with ethanol.

Benzyl alcohol is an item of commerce and, if used, is widely available from a number of sources (Remington's 17th Ed. p. 1057).

1,3-Propanediol, 2-amino-(2-hydroxymethyl)-(tromethamine) is available commercially (Remington's 17th Ed. p. 836). It can be made by additively reacting nitromethane with formaldehyde to yield tris (hydroxymethyl) nitromethane, and the nitro compound is then hydrogenated with Raney nickel in accordance with U.S. 2,174,242.

3-Mercapto-1,2-propanediol (monothioglycerol) is readily made, for example, by heating an ethanolic solution of 3-chloro-1,2-propanediol with potassium bisulfite (Remington's 17th Ed. p. 1279).

The amounts of the respective components can vary fairly broadly, within conventional limits well known to those skilled in this art. Preferred embodiments will be exemplified hereinafter. Typically the folic acid salt or leucovorin salt will comprise from about 0.5 to 50 mg/ml, preferably from about 1 to about 35 mg/ml and especially preferably from about 3 to about 25 mg/ml. With folic acid sodium salt, special mention is made of 5 mg/ml and for calcium leucovorin, 3 mg/ml.

Benzyl alcohol can be omitted, but if present, can comprise up to about 2.5 percent w/v, preferably up to 1.5 percent w/v with sodium folate and up to about 0.909 percent w/v with calcium leucovorin.

The amounts of tromethamine and monothioglycerol relative to each other can vary broadly, e.g., from about 1 to 99 parts by weight, preferably from about 20 to about 80 parts by weight of the former to from about 99 to 1 parts by weight, preferably from about 80 to about 20 parts of the latter. Preferably the tromethamine and monothioglycerol each will comprise from about 0.05 percent w/v to about 0.6 percent, preferably from about 0.1 to about 0.3 percent w/v of the composition. If sodium chloride is present, it can range from 0.1 to about 1.0 percent, preferably from about 0.45 to about 0.65 percent w/v. The pH adjustors can vary widely in type and amount. Typically hydrochloric acid, 5.0 percent v/v and sodium hydroxide 4.0% w/v will be conveniently employed.

The injectable solutions prepared as described above and more fully exemplified hereinafter are used in conventional dosages. A typical daily dose is generally up to about 150 mg, e.g., in the range of from about 25 to 150 mg which is conveniently administered in divided doses, for example 2, 3 or 4 doses in a 24 hour period (methotrexate rescue with calcium leucovorin injectable). For treating folate deficiency lower doses of leucovorin are generally administered. For example, a typical daily dose for an adult human is generally in the range of 2 to 25 mg which may be conveniently administered as a single dose (leucovorin calcium) or up to 1.0 mg daily (sodium folate).

### DESCRIPTION OF THE PREFERRED EMBODIMENTS

The following examples illustrate the invention.

In the data tables, the following abbreviations have the following meanings:
RT = room temperature
M = month
PABG = N-(p-aminobenzoyl)glutamic acid
FFA = (N¹⁰-formyl folic acid)
Leucovorin Potency = leucovorin calcium (as free acid)
%LP = percent of label potency
%L. = based on label potency of leucovorin
NMT = not more than
RHC = 37°C and 75% relative humidity in closed containers
SUN = exposure to sunlight
ND = none detected
RH = relative humidity
LCAB = light cabinet having 1,000 footcandle intensity
W = week

### EXAMPLE 1

Leucovorin calcium salt is used as the active ingredient in a composition for injection having the formula set forth in Table 1:

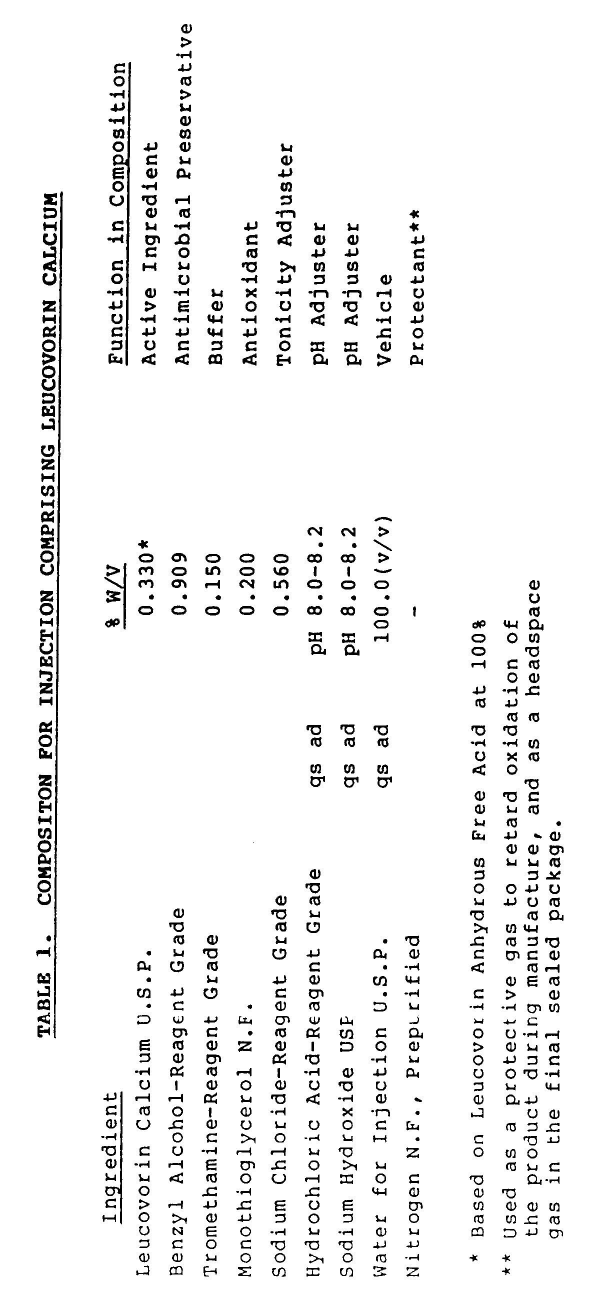

The formulation is prepared as follows:
A. Production of Bulk Solution
   Water for Injection, representing approximately 75% of final batch volume, is added to a stainless steel mixing tank. The Water for Injection is sparged with nitrogen until the water temperature has reached 25-30°C. (The product is continuously sparged or blanketed with nitrogen throughout the remaining process to protect against oxidation). The ingredients are sequentially added, mixed and dissolved in the following order: benzyl alcohol; sodium chloride; tromethamine; monothioglycerol; and calcium leucovorin. The pH is then adjusted to 8.1 ± 0.1 with 5% hydrochloric acid and/or 1% sodium hydroxide. The batch is brought to a final volume of 20-30 liters with Water for Injection sparged with nitrogen. The pH is re-checked and re-adjusted to 8.1 ± 0.1 with hydrochloric acid or sodium hydroxide if necessary.
B. Production of Sterile Filtrate
   Prior to the sterile filtration, a 0.2 micrometer filtration cartridge is tested for integrity by Bubble Point testing at a pressure of 30 psig. The bulk solution is then passed through a first stage pre-filtration unit containing an AW19/0.45 micrometer cartridge to an in-series sterile 0.2 micrometer second stage cartridge. Pump and Nitrogen gas pressure feed the solution through the filtration units to a tared sterile stainless steel collection drum. At the completion of filtration the 0.2 micrometer filtration unit is again tested for integrity by Bubble Point testing at 2.4 bar (35 psig). Elastomeric silicone tubing is used for all product transfer.
C. Production of Filled Product
   The bulk sterile solution is taken to a class 100 filling area where it is pumped from the collection drum through a sterile 5 micrometer filter to a filling line surge bottle. It is fed from the surge bottle to filling needles which eject measured doses to conveyor fed ampuls and vials. (Two filling lines, one for each package style, are used).

1cc Amber glass ampuls are filled at 1.15 ± 0.05 ml (1.0+0.15±0.05 ml USP overage). 10 ml (10 cc) Type 1 amber glass vials are filled at 10.5 ml (10.0 + 0.5 ml USP overage). All ampul and vial fills receive a nitrogen headspace blanket prior to sealing. Ampuls are heat sealed in a gas flame, and vials are sealed with butyl closures and aluminum crimp seals.

### EXAMPLES 2 AND 3

A second batch (Example 2) corresponding to Example 1 is prepared by the method described in Example 1 as in Table 1. A third batch (Example 3) is prepared, but this is 30 liters in size instead of 20 liters, as shown in Table 1.

### COMPARATIVE EXAMPLE 1A

A formulation like Example 1 without tromethamine and monothioglycerol having the formula set forth in Table 2 is included for comparison purposes:

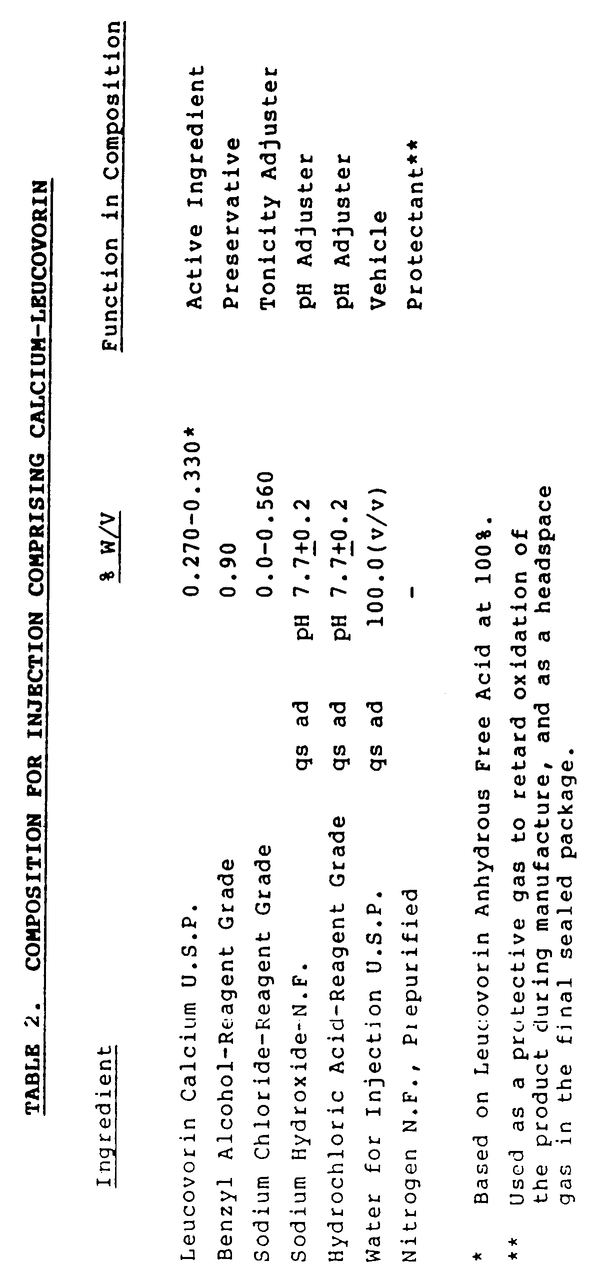

The general procedure of Example 1 is used to make filled dosage forms comprising the following:

| Batch | 1A | 2A | 3A | 4A |
|---|---|---|---|---|
| Ingredient | % w/v | % w/v | % w/v | % w/v |
| Calcium Leucovorin (As Free Acid) | 0.30 | 0.27 | 0.27 | 0.33 |
| Benzyl Alcohol Reagent | 0.90 | 0.90 | 0.90 | 0.90 |
| Sodium Chloride | - | - | 0.56 | 0.56 |
| Sodium Hydroxide 1.0% Solution | q.s. | q.s. | q.s. | q.s. |
| Hydrochloric Acid 1.0% Solution-Adjust to pH 7.7 | q.s. | q.s. | q.s. | q.s. |
| Water for Injection qs ad | 100 | 100 | 100 | 100 |

Amber glass ampules are the containers selected for filling.

### COMPARATIVE EXAMPLES 2A, 3A and 4A

A second, third, and fourth batch of formulae without tromethamine and monothioglycerol are prepared for comparative testing using the general procedure for Example 1.

The filled dosage forms from Examples 1-3 are placed on an accelerated stability test. The analytical methodolgy is as follows:

| **Analytical Methodology used for Examples 1-3** | |
|---|---|
| Test | Method |
| Description | visual |
| Leucovorin potency | High pressure liquid chromatography (HPLC) |
| Leucovorin related compounds potencies | (HPLC) |
| Benzyl Alcohol | Gas Chromatography |
| pH | pH meter |
| USP Particulate Matter | USP XXI Particulate Matter, Injections, light obscuration |
| Monothioglycerol (Identification) | Gas Chromotography |
| Microbiological evaluation of preservative system | Antimicrobial Preservative Effectiveness test USP XXI pp 1151-1156. |

The results of the tests performed on the packaged formulations of Examples 1-3 are reported in Tables 3, 4 and 5, respectively:

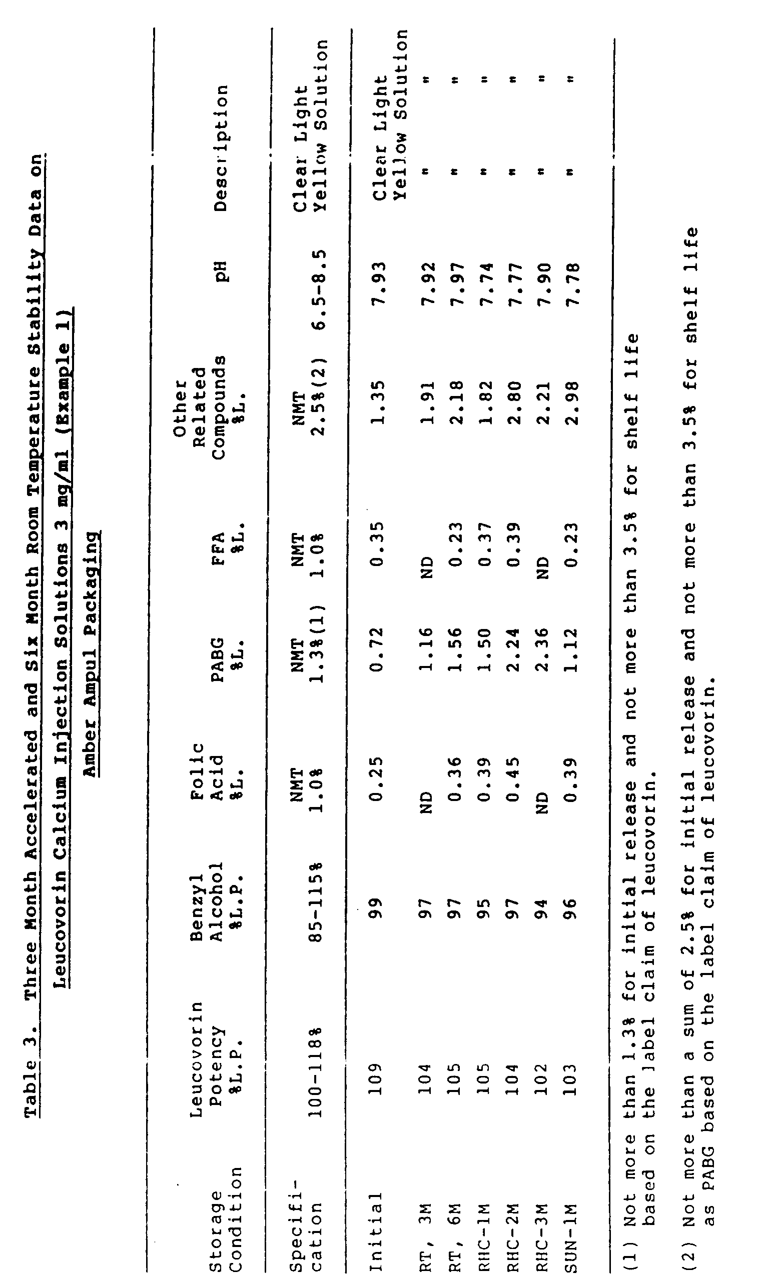

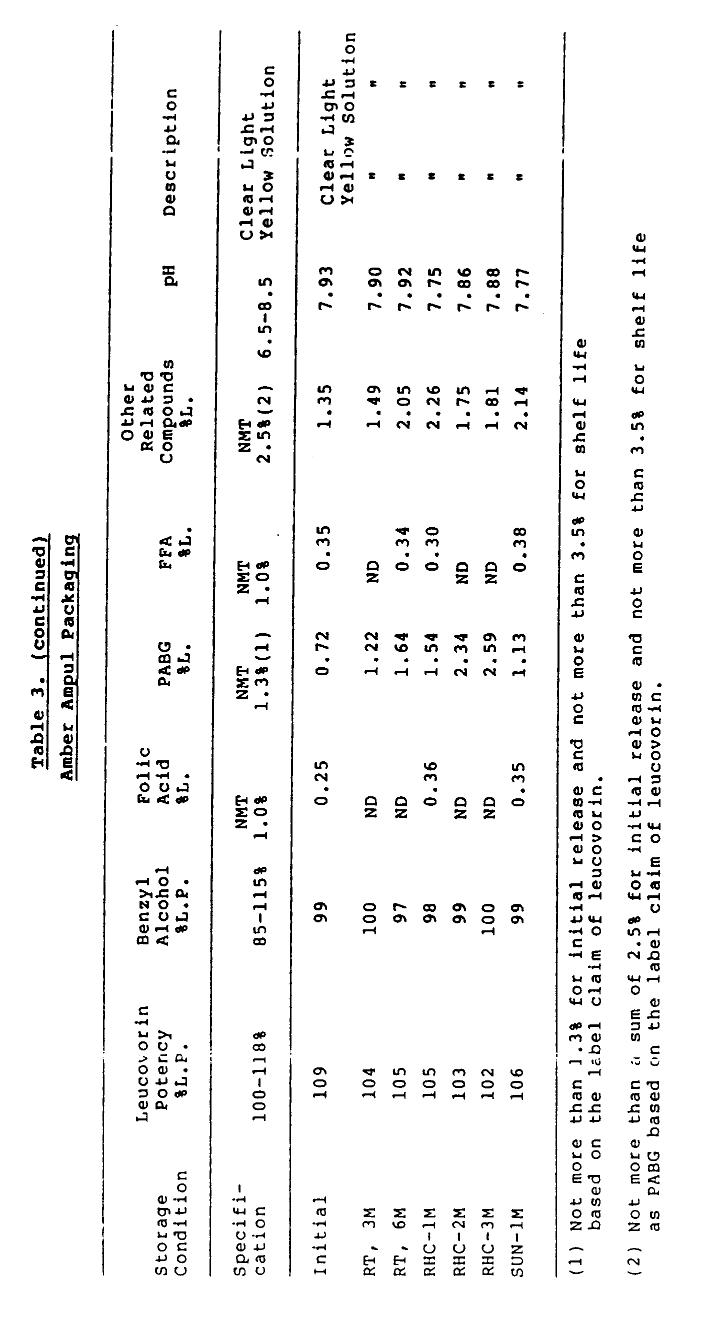

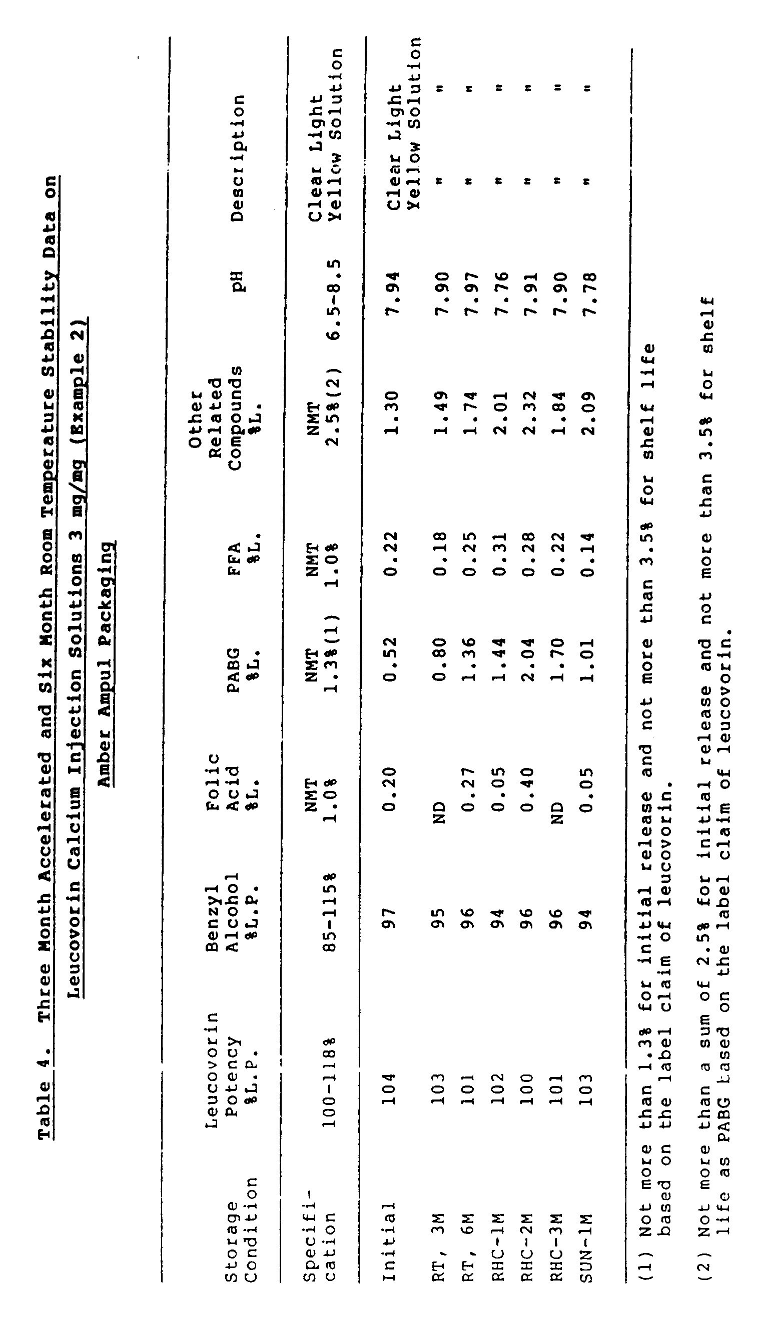

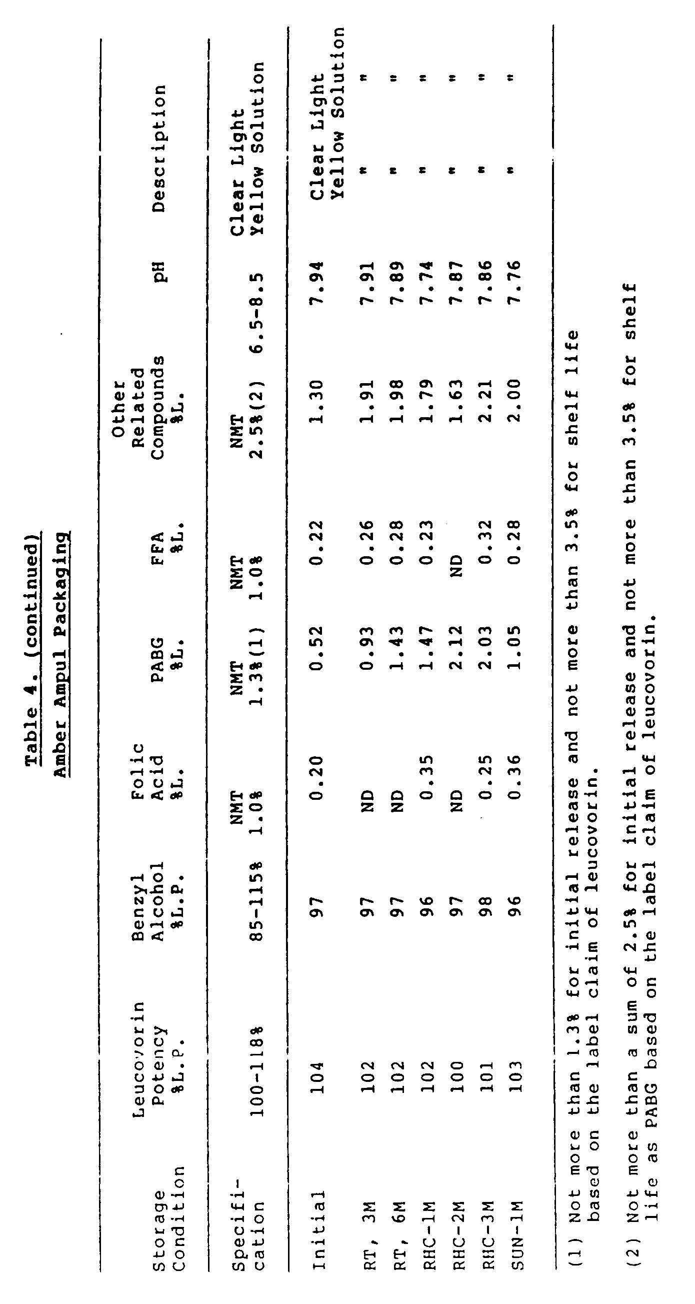

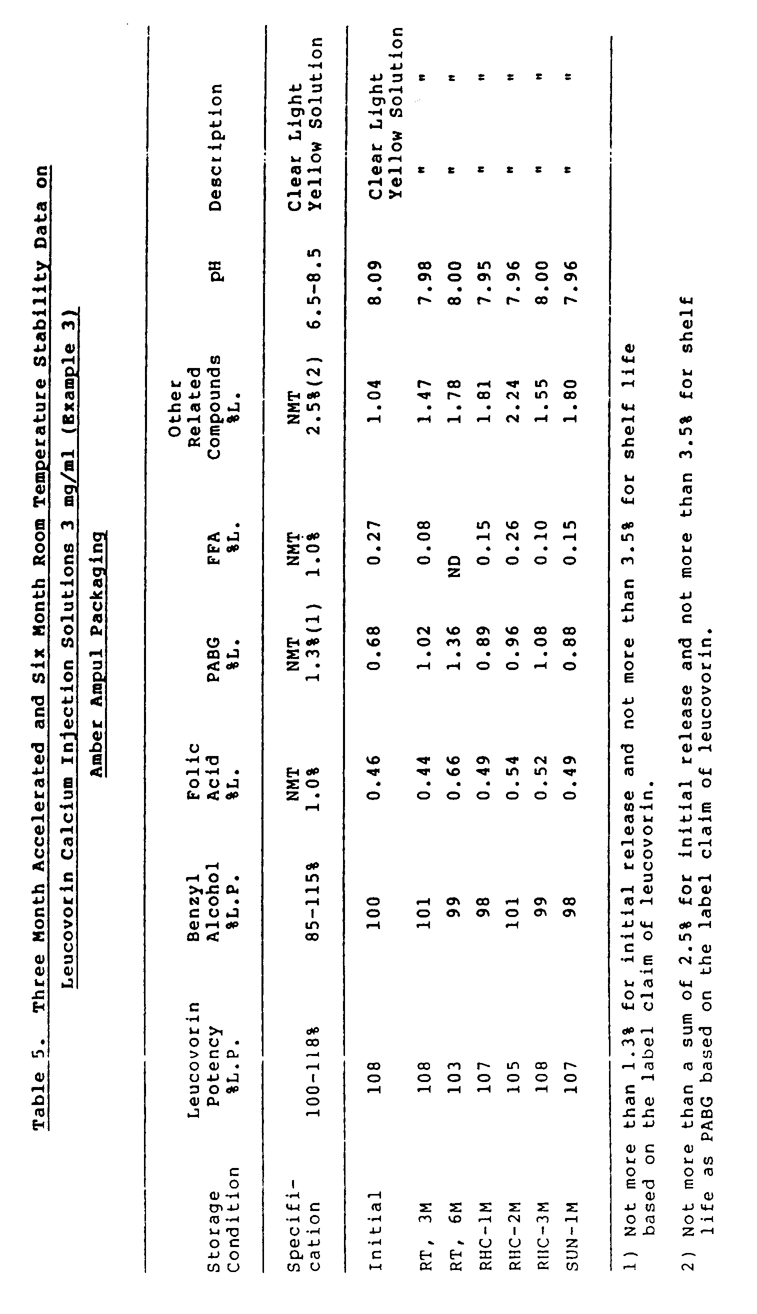

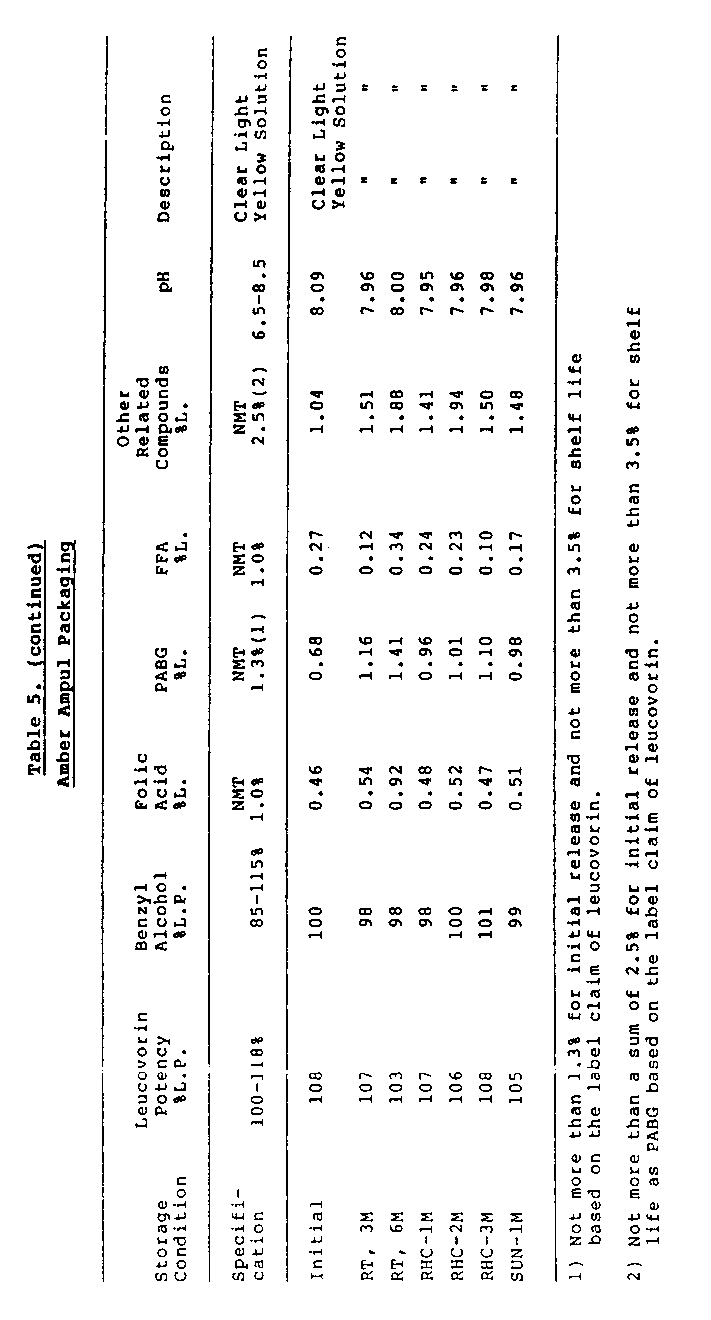

The effectiveness of the preservative system of this invention for Leucovorin Calcium Injectable Solutions, Examples 1-3, is tested in accordance with the Antimicrobial Preservatives Effectiveness Test outlined in U.S. Phamacopeia XXI, pages 1151-1156. This provides a measure of the capacity of the solutions to decrease microbial growth when individually challenged with Staphylococcus aureus, Escherichia coli, Psuedomonas aeruginusa, Candida albicans and Aspergillus niger at a dose level of 100,000 to 1,000,000 microorganisms per ml. The contaminated solutions are stored and sampled at a series of time intervals in order to obtain microorganism counts. The results of these tests, set forth in Tables 6, 7 and 8, indicate that the benzyl alcohol prevents the growth of microorganisms, and provides qualities necessary to pass the anti-microbial preservative effectiveness test.

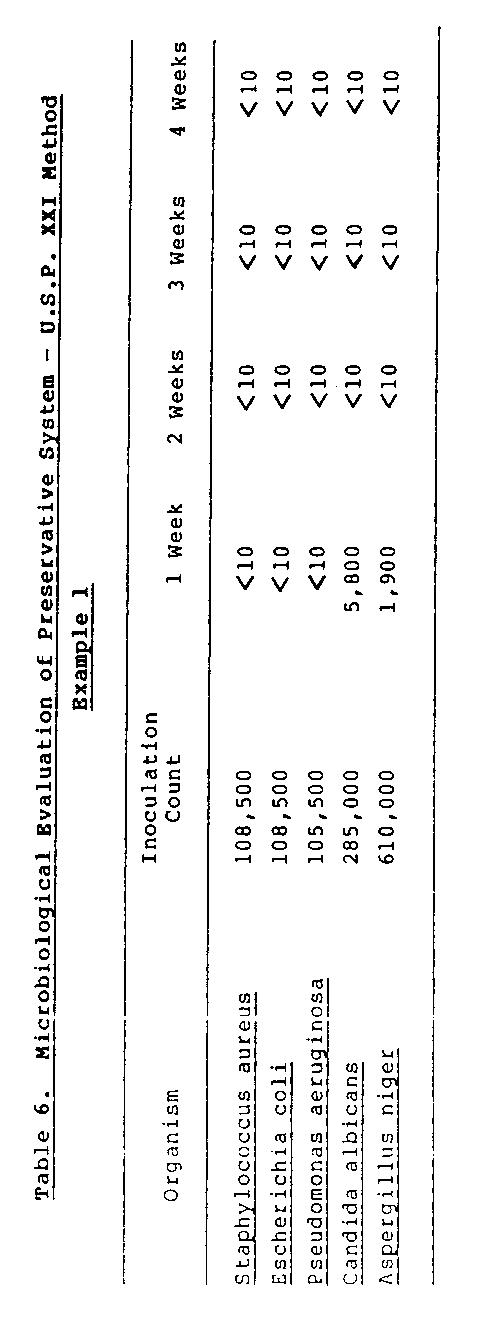

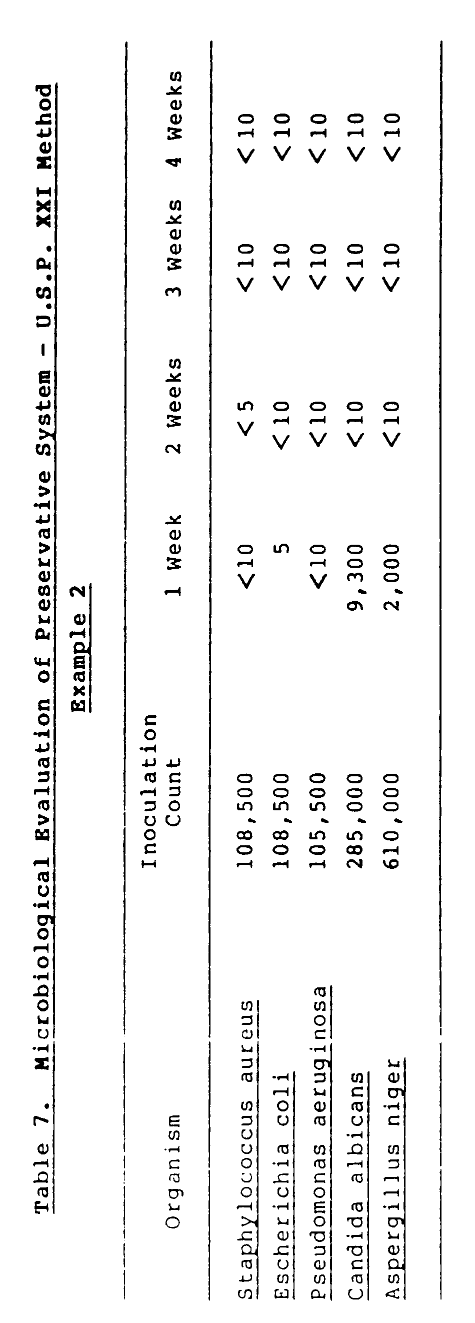

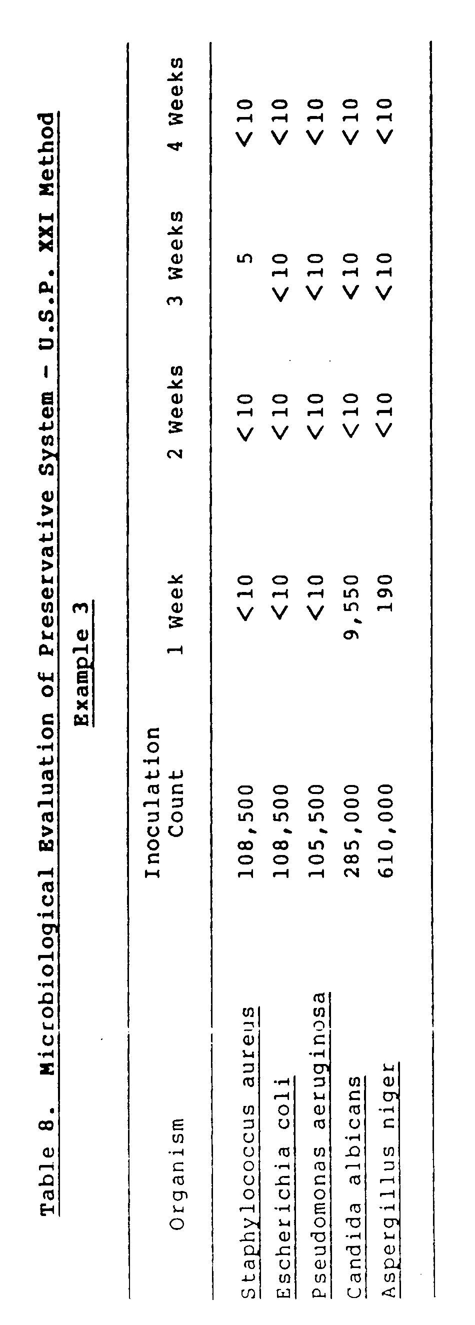

The storage stability and microbiological testing of the injectable leucovorin calcium preparations set forth under Comparative Examples 1A, 1B, 1C and 1D using standard test methods are determined by the following procedures:

| Test | Method |
|---|---|
| Description | Visual |
| Leucovorin potency | Thin layer Chromatography |
| Benzyl Alcohol | Spectrophotometric Gas Liquid Chromatography |
| pH | pH meter |
| Microbiological evaluation of preservative system | Antimicrobial Effectiveness Test USP XIX page 587 |

Stability data for the comparison calcium injection compositions are set forth in Tables 9, 10 and 11:

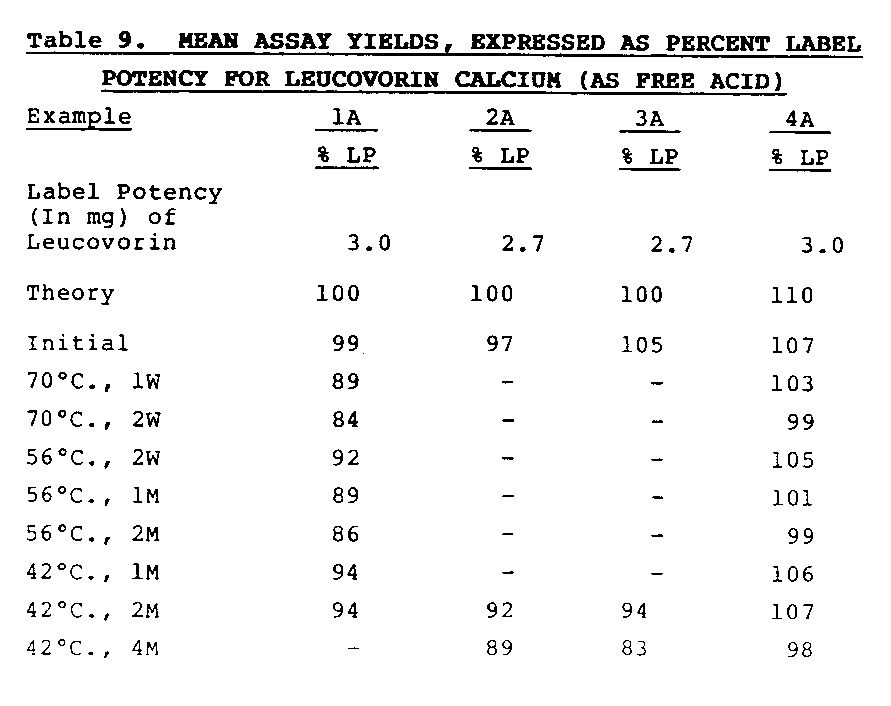

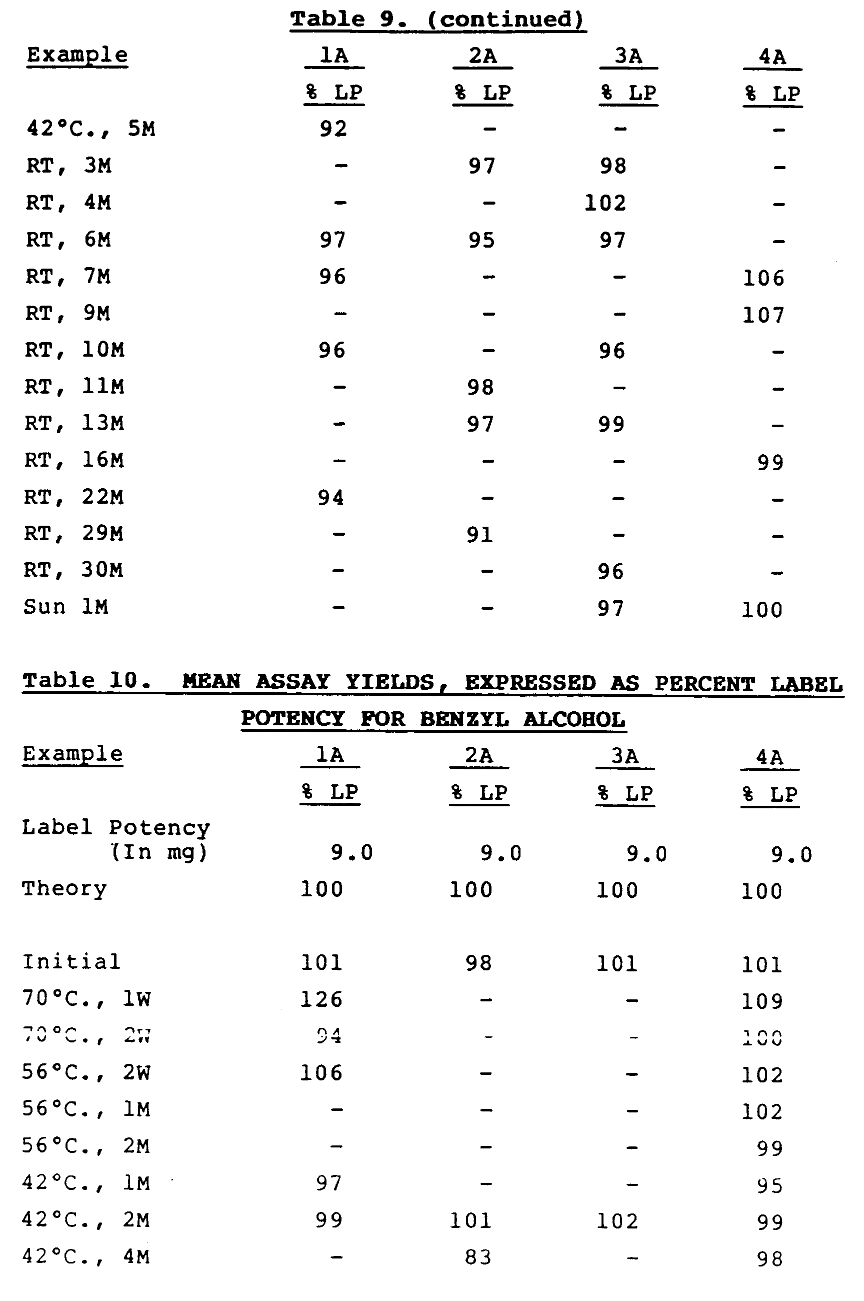

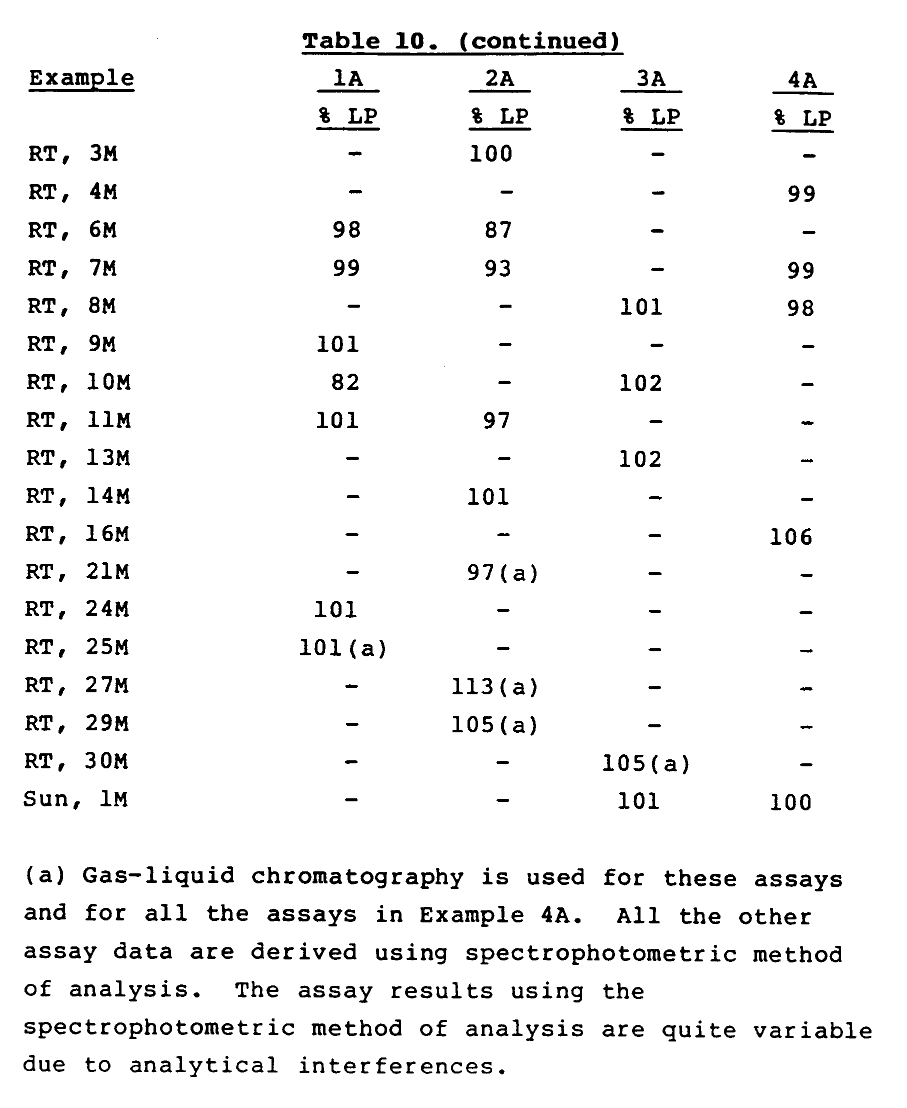

**Table 11**

| **ELECTROMETRIC pH DETERMINATIONS AT AMBIENT TEMPERATURE** | | | | |
|---|---|---|---|---|
| | pH Value | | | |
| Example | 1A | 2A | 3A | 4A |
| Initial | 7.7 | 7.7 | 7.7 | 7.7 |
| RT, 2M | - | 6.6 | - | 6.5 |
| RT, 4M | - | 7.0 | - | - |
| RT, 6M | 6.8 | - | - | 6.6 |
| RT, 9M | 6.4 | - | 6.8 | - |
| RT, 12M | - | - | - | 6.5 |
| RT, 24M | - | 6.7 | - | - |
| RT, 28M | - | - | 7.0 | - |
| RT, 29M | - | 6.5 | - | - |
| RT, 32M | - | - | - | 6.4 |
| RT, 34M | 6.6 | - | - | - |
| RT, 36M | - | - | - | 6.2 |

The effectiveness of the preservative system of Leucovorin Calcium Injectable Solutions, for Comparative Examples 1A, 2A, 3A, and 4A is tested in accordance with the Antimicrobial Preservatives Effectiveness Test outlined measure of the capacity of the solutions to decrease microbial growth when individually challenged with Staphylococcus aureus, Escherichia coli, Psuedomonas aeruginusa, Candid albicans and Aspergillus niger at a dose level of 100,000 to 1,000,000 microorganisms per ml. The contaminated solutions are stored and sampled at a series of time intervals in order to obtain microorganism counts. The results of these tests are set forth in Table 12.

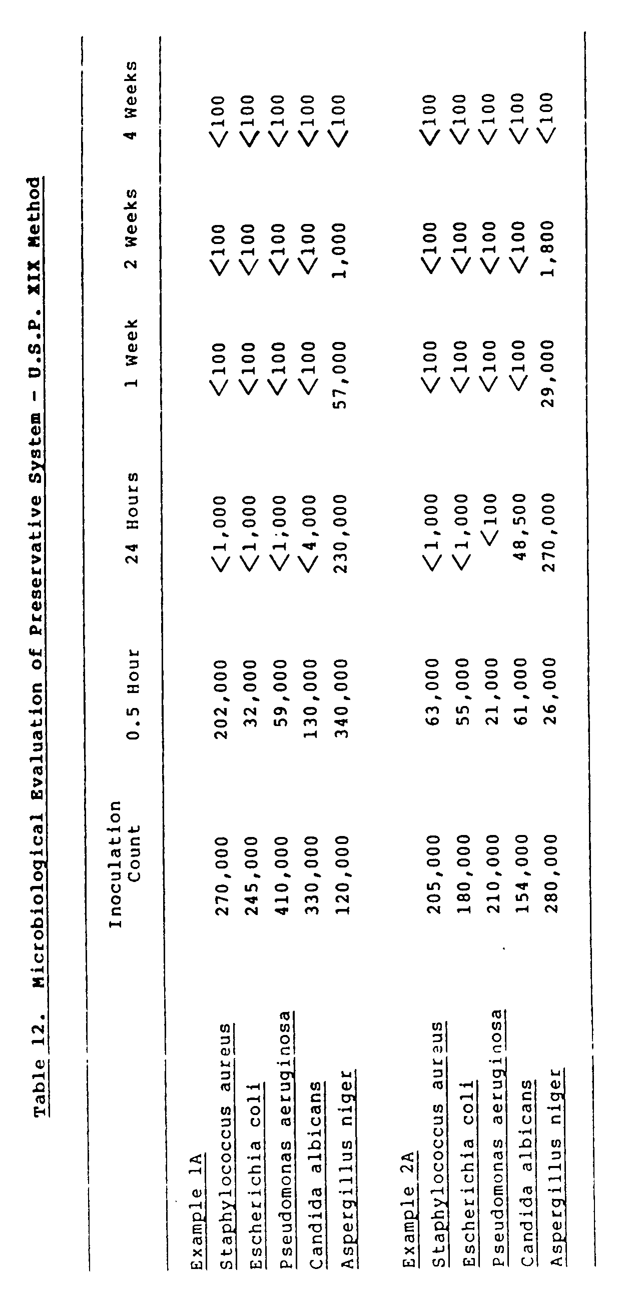

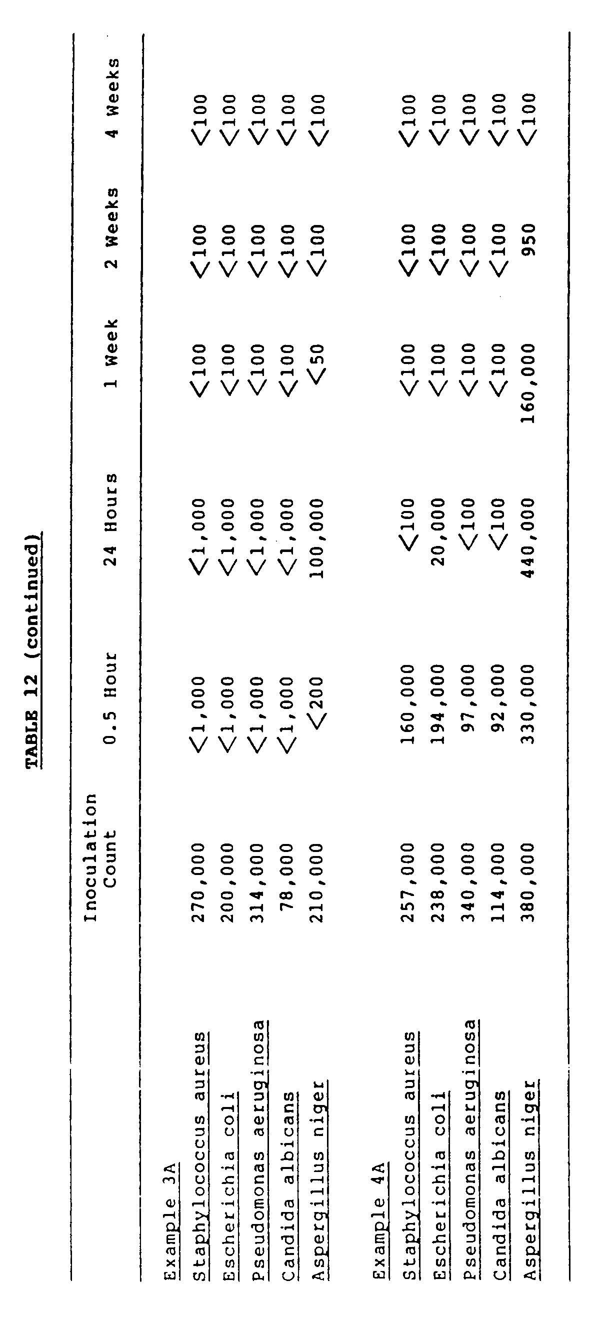

### EXAMPLES 4, 5 AND 6

Three batches are prepared with leucovorin calcium salt as the active ingredient in a composition for injection having the formula set forth in Table 13:

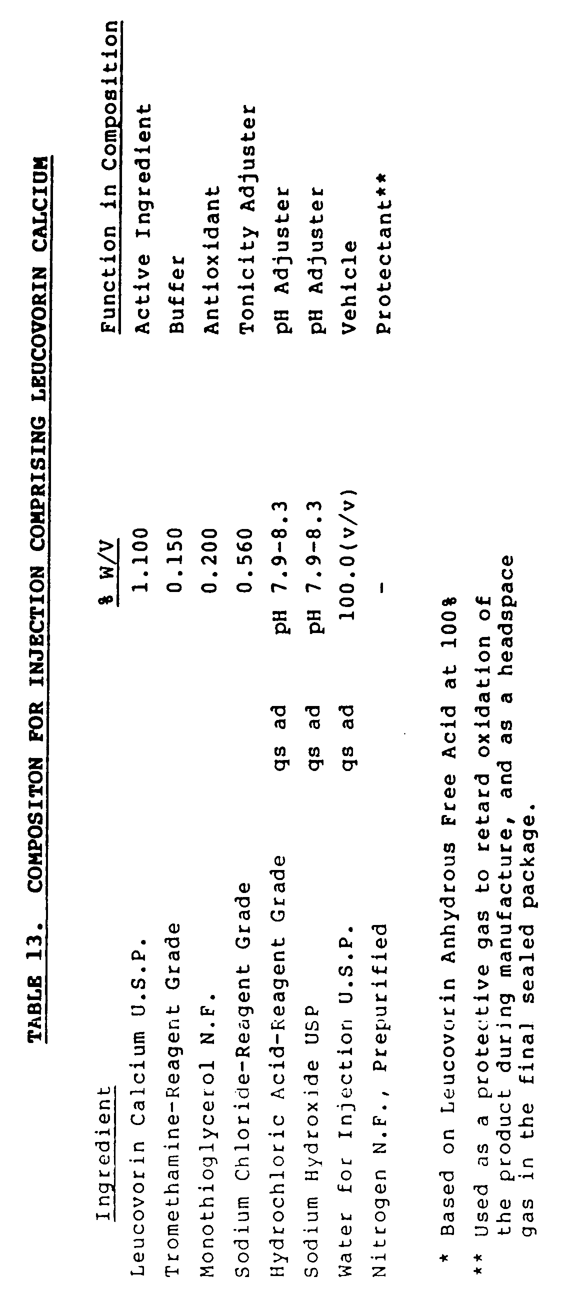

The resulting composition is a clear light yellow solution. Each batch is 40 liters. The solutions are then distributed to 50 ml amber tubing vials with 50 mg/vial label potency. Grey butyl plug closures are used with aluminum seals.

The filled dosage forms from Examples 4, 5 and 6 are placed on accelerated stability tests, consisting of storing the solution at 23°C for up to 9 months, storing the solution at 40°C and at 75% relative humidity for up to three months, and storing the solution for one month in a light cabinet.

The solutions are assayed by high performance liquid chromatography. The mean assay results of the tests performed on the packaged formulations of Examples 4, 5 and 6 are reported in Table 14:

**Table 14**

| **Mean Assay Results For Leucovorin Calcium Injection Solutions 500 mg/vial (Examples 4, 5 and 6)** | | | | |
|---|---|---|---|---|
| Example No. | | 4 | 5 | 6 |
| Storage Condition | Time | % L.P. | % L.P. | % L.P. |
| Initial | | 110 | 106 | 109 |
| 23°C | 3M | 106 | 104 | 106 |
| 23°C | 6M | 107 | 105 | 106 |
| 23°C | 9M | 106 | 104 | 105 |
| 23°C | 12M | 103 | 102 | 104 |
| 40°C/75% RH | 1M | 102 | 101 | 101 |
| 40°C/75% RH | 2M | 99 | 98 | 100 |
| 40°C/75% RH | 3M | 98 | 97 | 99 |
| LCAB* | 1M | 107 | 105 | 106 |

The packaged formulations of Examples 4, 5 and 6 did not change in appearance after being stored at 23°C for up to 9 months, 40°C and 75% relative humidity for up to three months, and in a light cabinet for one month. The compositions remained clear light yellow solutions.

The solutions of Examples 4, 5 and 6 are measured electrometrically for pH. The results of these tests, showing that the pH remained essentially unchanged at all storage conditions, are reported in Table 15:

**TABLE 15**

| **pH Measurement Data For Leucovorin Calcium Injection Solutions 500 mg/vial (Examples 4, 5 and 6)** | | | | |
|---|---|---|---|---|
| Example No. | | 4 | 5 | 6 |
| Storage Condition | Time | pH | pH | pH |
| Initial | | 8.2 | 8.4 | 8.4 |
| 23°C | 3M | 8.1 | 8.2 | 8.3 |
| 23°C | 6M | 8.0 | 8.1 | 8.2 |
| 23°C | 9M | 8.1 | 8.3 | 8.4 |
| 23°C | 12M | 8.1 | 8.2 | 8.3 |
| 40°C/75% RH | 1M | 8.2 | 8.4 | 8.4 |
| 40°C/75% RH | 2M | 8.0 | 7.9 | 8.1 |
| 40°C/75% RH | 3M | 8.0 | 8.0 | 8.1 |

### Example 5

If the procedure of Example 1 is repeated substituting sodium folate for the calcium leucovorin, using the following ingredients: sodium folate equivalent to 5 mg of folic acid; disodium edetate 0.2%; Water for Injection; q.s. 100%; sodium hydroxide to approximately pH 9; benzyl alcohol; 1.5%; tromethamine, 0.15%; and monothioglycerol, 0.200%, all percentages by weight over volume, a stable injectable composition in accordance with this invention will be obtained.

The foregoing data indicate that a unique blend of a buffer and an antioxidant endows leucovorin with more stability than the present standard formulation of leucovorin (Comparative Examples). More particularly, the composition of this invention is shown to be:
(1) a formulation which is more stable than the present standard product system in terms of pH: with variations of no more than 0.4 pH units from an adjusted pH of 8.1, and thus provides a stabilized alkaline pH to insure maximum stability of leucovorin calcium;
(2) a formulation with better leucovorin potency photostability after one month exposure to sunlight when compared to the standard product formulation;
(3) a formulation which has equivalent or better leucovorin potency stability overall;
(4) a formulation with equivalent benzyl alcohol potency stability, as compared to the standard product formulation.
(5) a formulation where the generation of related compounds does not exceed the specified limits for the standard product formulation;
(6) a formulation which meets the present Antimicrobial Preservatives Effectiveness test as outlined in U.S.P. XXI; and
(7) a formulation which has a projected expiration dating of at least eighteen months.

The foregoing detailed description will suggest many obvious variations to those skilled in this art. For example, instead of calcium leucovorin, strontium leucovorin and sodium leucovorin can be used. Racemic and enantiomeric l-leucovorin can be used. Benzyl alcohol can be omitted. Instead of sodium folate, calcium folate can be used. Derivatives, such as polyglutamyl folates and leucovorin can be used. The injectable compositions can contain conventional additives such as chelating agents, inert gases, and the like. All such obvious modifications are within the full intended scope of the appended Claims.

## Claims (Claims for the following Contracting State(s): AT, BE, CH, DE, DK, FR, GB, IT, LI, NL, SE)

1. A stable, injectable aqueous composition comprising:
(i) an effective amount of a water soluble pharmaceutically-acceptable salt of folic acid or leucovorin; optionally,
(ii) an effective preservative amount of benzyl alcohol; and
(iii) an effective amount of a buffer/antioxidant combination comprising
(a) tromethamine and
(b) monothioglycerol, said combination (iii) being present in an amount at least sufficient to maintain the pH of said composition in a predetermined range of from about 6 to about 10 and to protect the composition against degradation induced by oxygen or light.

2. A composition as defined in Claim 1, wherein said salt comprises a salt of leucovorin.

3. A composition as defined in Claim 2, wherein said leucovorin salt comprises calcium leucovorin.

4. A composition as defined in Claim 2, wherein said salt comprises a salt of l-leucovorin.

5. A pharmaceutical formulation comprising from 3 mg/ml to 25 mg/ml of Leucovorin Calcium in an isotonic solution at a pH of from 6.5 to 8.5, with or without benzyl alcohol at a concentration of from 0.0% w/v to 0.909% w/v, TRIS (tromethamine) at a concentration of from 0.10% w/v to 0.30% w/v, monothioglycerol at a concentration of from 0.10% w/v to 0.30% w/v; sodium chloride at a concentration from 0.45% to 0.65% w/v, with hydrochloric acid at a concentration of 5.0 v/v% to 10.0 v/v% and sodium hydroxide at a concentration of 1.0 w/v% to 5.0 w/v% added as required to adjust the pH.

6. A method for stabilizing an injectable aqueous composition against deterioration, said method comprising:
A. providing an aqueous composition comprising
(i) an effective amount of a water- soluble pharmaceutically acceptable salt of folic acid or leucovorin and, optionally,
(ii) a small effective preservative amount of benzyl alcohol, and
B. adding thereto
(iii) an effective amount of a buffer/antioxidant combination comprising
(a) tromethamine and
(b) monothioglycerol, said combination being provided in an amount at least sufficient to maintain the pH of said composition in a predetermined range of from about 6 to about 10 and to protect the composition against degradation induced by oxygen and light.

7. A method as defined in Claim 6, wherein said salt comprises a salt of leucovorin.

8. A method as defined in Claim 6, wherein said salt comprises a salt of l-leucovorin.

9. A method for stabilizing a pharmaceutical formulation comprising providing from 3 mg/ml to 25 mg/ml of Leucovorin Calcium in an insotonic solution at a pH of from 6.5 to 8.5, with or without benzyl alcohol at a concentration of from 0.0% w/v to 0.909% w/v, and adding thereto TRIS (tromethamine) at a concentration from 0.10% w/v to 0.30% w/v, monothioglycerol at a concentration of from 0.10% w/v to 0.30% w/v; sodium chloride at a concentration of from 0.45% w/v to 0.65% w/v, and sufficient hydrochloric acid at a concentration of 5.0 v/v% to 10.0 v/v% and sodium hydroxide at a concentration of 1.0 w/v% to 5.0% w/v added as needed to adjust the pH.

## Claims (Claims for the following Contracting State(s): ES, GR)

1. A method for stabilizing an injectable aqueous composition against deterioration, said method comprising:
A. providing an aqueous composition comprising
(i) an effective amount of a water- soluble pharmaceutically acceptable salt of folic acid or leucovorin and, optionally,
(ii) a small effective preservative amount of benzyl alcohol, and
B. adding thereto
(iii) an effective amount of a buffer/antioxidant combination comprising
(a) tromethamine and
(b) monothioglycerol, said combination being provided in an amount at least sufficient to maintain the pH of said composition in a predetermined range of from about 6 to about 10 and to protect the composition against degradation induced by oxygen and light.

2. A method as defined in Claim 1, wherein said salt comprises a salt of leucovorin.

3. A method as defined in Claim 1, wherein said salt comprises a salt of l-leucovorin.

4. A method for stabilizing a pharmaceutical formulation comprising providing from 3 mg/ml to 25 mg/ml of Leucovorin Calcium in an isotonic solution at a pH of from 6.5 to 8.5, with or without benzyl alcohol at a concentration of from 0.0% w/v to 0.909% w/v, and adding thereto TRIS (tromethamine) at a concentration from 0.10% w/v to 0.30% w/v, monothioglycerol at a concentration of from 0.10% w/v to 0.30% w/v; sodium chloride at a concentration of from 0.45% w/v to 0.65% w/v, and sufficient hydrochloric acid at a concentration of 5.0 v/v% to 10.0 v/v% and sodium hydroxide at a concentration of 1.0 w/v% to 5.0% w/v added as needed to adjust the pH.

## Patentansprüche (Patentansprüche für folgende(n) Vertragsstaat(en): AT, BE, CH, DE, DK, FR, GB, IT, LI, NL, SE)

1. Stabile, injizierbare, wässerige Zusammensetzung, umfassend:
(i) eine wirksame Menge eines wasserlöslichen, pharmazeutisch akzeptablen Salzes von Folsäure oder Leucovorin; gegebenenfalls
(ii) eine wirksame, haltbar machende Menge von Benzylalkohol und
(iii) eine wirksame Menge einer Kombination aus Puffer und Oxidationsmittel, umfassend
(a) Tromethamin und
(b) Monothioglycerin,
wobei die Kombination (iii) in einer Menge vorhanden ist, die zumindest genügt, um den pH der Zusammensetzung in einem vorbestimmten Bereich von etwa 6 bis etwa 10 zu halten und die Zusammensetzung gegen durch Sauerstoff oder Licht induzierten Abbau zu schützen.

2. Zusammensetzung nach Anspruch 1, worin das Salz ein Salz von Leucovorin umfaßt.

3. Zusammensetzung nach Anspruch 2, worin das Leucovorinsalz Calciumleucovorin umfaßt.

4. Zusammensetzung nach Anspruch 2, worin das Salz ein Salz von ℓ-Leucovorin umfaßt.

5. Pharmazeutische Formulierung, umfassend von 3 mg/mℓ bis 25 mg/mℓ des Calciumsalzes von Leucovorin in einer isotonen Losung bei einem pH von 6,5 bis 8,5 mit oder ohne Benzylalkohol bei einer Konzentration von 0 bis 0,909% Gew./Vol., TRIS(Tromethamin) in einer Konzentration von 0,10 bis 0,30% Gew./Vol., Monothioglycerin in einer Konzentration von 0,10 bis 0,30% Gew./Vol., Natriumchlorid in einer Konzentration von 0,45 bis 0,65% Gew./Vol., wobei Chlorwasserstoffsäure in einer Konzentration von 5,0 bis 10,0 Vol./Vol.-% und Natriumhydroxid in einer Konzentration von 1,0 bis 5,0 Gew./Vol.-% hinzugegeben sind, wie zum Einstellen des pH erforderlich.

6. Verfahren zum Stabilisieren einer injizierbaren, wässerigen Zusammensetzung gegen Abbau, wobei das Verfahren umfaßt:
A. Herstellen einer wässerigen Zusammensetzung, umfassend
(i) eine wirksame Menge eines wasserlöslichen, pharmazeutisch akzeptablen Salzes von Folsäure oder Leucovorin und, gegebenenfalls,
(ii) eine geringe wirksame, haltbar machende Menge von Benzylalkohol und
B. Hinzugeben von
(iii) einer wirksamen Menge einer Kombination aus Puffer und Antioxidationsmittel, umfassend
(a) Tromethamin und
(b) Monothioglycerin,
wobei diese Kombination in einer Menge hnzugegeben wird, die mindestens genügt, um den pH der Zusammensetzung in einem vorbestimmten Bereich von etwa 6 bis etwa 10 zu halten und die Zusammensetzung gegen durch Sauerstoff und Licht induzierten Abbau zu schützen.

7. Verfahren nach Anspruch 6, worin das Salz ein Salz von Leucovorin umfaßt.

8. Verfahren nach Anspruch 6, worin das Salz ein Salz von ℓ-Leucovorin umfaßt.

9. Verfahren zum Stabilisieren einer pharmazeutischen Formulierung, umfassend Schaffen von 3 mg/mℓ bis 25 mg/mℓ des Calciumsalzes von Leucovorin in einer isotonen Lösung bei einem pH von 6,5 bis 8,5 mit oder ohne Benzylalkohol bei einer Konzentration von 0 bis 0,909% Gew./Vol., TRIS(Tromethamin) in einer Konzentration von 0,10 bis 0,30% Gew./Vol., Monothioglycerin in einer Konzentration von 0,10 bis 0,30% Gew./Vol., Natriumchlorid in einer Konzentration von 0,45 bis 0,65% Gew./Vol., und genügend Chlorwasserstoffsäure in einer Konzentration von 5,0 bis 10,0 Vol./Vol.-% und Natriumhydroxid in einer Konzentration von 1,0 bis 5,0 Gew./Vol.-% hinzugegeben werden, wie zum Einstellen des pH erforderlich.

## Patentansprüche (Patentansprüche für folgende(n) Vertragsstaat(en): ES, GR)

1. Verfahren zum Stabilisieren einer injizierbaren, wässerigen Zusammensetzung gegen Abbau, wobei das Verfahren umfaßt:
A. Herstellen einer wässerigen Zusammensetzung, umfassend
(i) eine wirksame Menge eines wasserlöslichen, pharmazeutisch akzeptablen Salzes von Folsäure oder Leucovorin und, gegebenenfalls,
(ii) eine geringe wirksame, haltbar machende Menge von Benzylalkohol und
B. Hinzugeben von
(iii) einer wirksamen Menge einer Kombination aus Puffer und Antioxidationsmittel, umfassend
(a) Tromethamin und
(b) Monothioglycerin,
wobei diese Kombination in einer Menge hnzugegeben wird, die mindestens genügt, um den pH der Zusammensetzung in einem vorbestimmten Bereich von etwa 6 bis etwa 10 zu halten und die Zusammensetzung gegen durch Sauerstoff und Licht induzierten Abbau zu schützen.

2. Verfahren nach Anspruch 1, worin das Salz ein Salz von Leucovorin umfaßt.

3. Verfahren nach Anspruch 1, worin das Salz ein Salz von ℓ-Leucovorin umfaßt.

4. Verfahren zum Stabilisieren einer pharmazeutischen Formulierung, umfassend Schaffen von 3 mg/mℓ bis 25 mg/mℓ des Calciumsalzes von Leucovorin in einer isotonen Lösung bei einem pH von 6,5 bis 8,5 mit oder ohne Benzylalkohol bei einer Konzentration von 0 bis 0,909% Gew./Vol., und Hinzugeben von TRIS(Tromethamin) in einer Konzentration von 0,10 bis 0,30% Gew./Vol., Monothioglycerin in einer Konzentration von 0,10 bis 0,30% Gew./Vol., Natriumchlorid in einer Konzentration von 0,45 bis 0,65% Gew./Vol., und von genügend Chlorwasserstoffsäure in einer Konzentration von 5,0 bis 10,0 Vol./Vol.-% und Natriumhydroxid in einer Konzentration von 1,0 bis 5,0 Gew./Vol.-%, wie zum Einstellen des pH erforderlich.

## Revendications (Revendications pour l'(les) Etat(s) contractant(s) suivant(s): AT, BE, CH, DE, DK, FR, GB, IT, LI, NL, SE)

1. Composition aqueuse stable injectable comprenant ;
(i) une quantité efficace d'un sel d'acide folique ou de leucovorine hydrosoluble acceptable du point de vue pharmaceutique ; éventuellement,
(ii) une quantité efficace du point de vue de la conservation d'alcool benzylique ; et
(iii) une quantité efficace d'une combinaison tampon/anti-oxydant comprenant :
(a) de la trométhamine et
(b) du monothioglycérol, ladite combinaison (iii) étant présente en une quantité au moins suffisante pour maintenir le pH de ladite composition dans un intervalle prédéterminé d'environ 6 à environ 10 et pour protéger la composition contre la dégradation induite par l'oxygène ou la lumière.

2. Composition telle que définie dans la revendication 1, dans laquelle ledit sel comprend un sel de leucovorine.

3. Composition telle que définie dans la revendication 2, dans laquelle ledit sel de leucovorine comprend de la leucovorine de calcium.

4. Composition telle que définie dans la revendication 2, dans laquelle ledit sel comprend un sel de l-leucovorine.

5. Formulation pharmaceutique comprenant de 3 mg/ml à 25 mg/ml de Leucovorine Calcium dans une solution isotonique à un pH compris entre 6,5 et 8,5, avec ou sans alcool benzylique à une concentration comprise entre 0,0% p/v et 0,909% p/v, du TRIS (trométhamine) à une concentration comprise entre 0,10% p/v et 0,30% p/v, du monothioglycérol à une concentration comprise entre 0,10% p/v et 0,30% p/v ; du chlorure de sodium à une concentration comprise entre 0,45% et 0,65% p/v, avec de l'acide chlorydrique à une concentration comprise entre 5,0% v/v et 10,0% v/v et de l'hydroxyde de sodium à une concentration comprise entre 1,0 p/v% et 5,0% p/v ajouté si nécessaire pour ajuster le pH.

6. Procédé de stabilisation d'une composition aqueuse injectable contre la détérioration, ledit procédé comprenant les étapes de :
A. fourniture d'une composition aqueuse stable injectable comprenant :
(i) une quantité efficace d'un sel d'acide folique ou de leucovorine hydrosoluble acceptable du point de vue pharmaceutique ; éventuellement,
(ii) une quantité efficace du point de vue de la conservation d'alcool benzylique ; et
B. addition à celle-ci de
(iii) une quantité efficace d'une combinaison tampon/anti-oxydant comprenant :
(a) de la trométhamine et
(b) du monothioglycérol, ladite combinaison (iii) étant présente en une quantité au moins suffisante pour maintenir le pH de ladite composition dans un intervalle prédéterminé d'environ 6 à environ 10 et pour protéger la composition contre la dégradation induite par l'oxygène et la lumière.

7. Procédé tel que défini dans la revendication 6, dans lequel ledit sel comprend un sel de leucovorine.

8. Procédé tel que défini dans la revendication 6, dans lequel ledit sel de leucovorine comprend un sel de l-leucovorine.

9. Procédé de stabilisation d'une formulation pharmaceutique comprenant la fourniture de 3 mg/ml à 25 mg/ml de Leucovorine Calcium dans une solution isotonique à un pH compris entre 6,5 et 8,5, avec ou sans alcool benzylique à une concentration comprise entre 0,0% p/v et 0,909% p/v l'addition à celle-ci de TRIS (trométhamine) à une concentration comprise entre 0,10% p/v et 0,30% p/v, de monothioglycérol à une concentration comprise entre 0,10% p/v et 0,30% p/v ; de chlorure de sodium à une concentration comprise entre 0,45% et 0,65% p/v, et suffisamment d'acide chlorydrique à une concentration comprise entre 5,0% v/v et 10,0% v/v et d'hydroxyde de sodium à une concentration comprise entre 1,0 p/v% et 5,0% p/v, ajoutés si nécessaire pour ajuster le pH.

## Revendications (Revendications pour l'(les) Etat(s) contractant(s) suivant(s): ES, GR)

1. Procédé de stabilisation d'une composition aqueuse injectable contre la détérioration, ledit procédé comprenant les étapes de :
A. Fourniture d'une composition aqueuse comprenant :
(i) une quantité efficace d'un sel d'acide folique ou de leucovorine hydrosoluble acceptable du point de vue pharmaceutique et éventuellement,
(ii) une faible quantité efficace du point de vue de la conservation d'alcool benzylique ; et
B. addition à celle-ci :
(iii) d'une quantité efficace d'une combinaison tampon/anti-oxydant comprenant :
(a) de la trométhamine et
(b) du monothioglycérol, ladite combinaison étant présente en une quantité au moins suffisante pour maintenir le pH de ladite composition dans un intervalle prédéterminé d'environ 6 à environ 10 et pour protéger la composition contre la dégradation induite par l'oxygène et la lumière.

2. Procédé tel que défini dans la revendication 1, dans lequel ledit sel comprend un sel de leucovorine.

3. Procédé tel que défini dans la revendication 1, dans lequel ledit sel de leucovorine comprend un sel de l-leucovorine.

4. Procédé de stabilisation d'une formulation pharmaceutique comprenant la fourniture de 3 mg/ml à 25 mg/ml de Leucovorine Calcium dans une solution isotonique à un pH compris entre 6,5 et 8,5 avec ou sans alcool benzylique à une concentration comprise entre 0,0 % p/v et 0,909 % p/v, et l'addition à celle-ci de TRIS (trométhamine) à une concentration comprise entre 0,10 % p/v et 0,30 % p/v, de monothioglycérol à une concentration comprise entre 0,10 % p/v et 0,30 % p/v, de chlorure de sodium à une concentration comprise entre 0,45 % et 0,65 % p/v, et suffisamment d'acide chlorhydrique à une concentration comprise entre 5,0 % v/v et 10,0 % v/v et d'hydroxyde de sodium à une concentration comprise entre 1,0 % p/v et 5,0 % p/v, ajoutés si nécessaire pour ajuster le pH.
